# FASCICULE DE BREVET EUROPEEN

(11) **EP 0 577 529 B1**
(45) Date de publication et mention de la délivrance du brevet: **16.10.1996**
(21) Numéro de dépôt: 93420268.0
(22) Date de dépôt: 23.06.1993
(51) Int. Cl.: A61F 2/38

(54) **Prothèse totale du genou, du type à glissement**
Totale Gleitprothese für das Knie
Total sliding prosthesis for the knee

(30) Priorité: 23.06.1992 FR 9208055
(43) Date de publication de la demande: 05.01.1994
(73) Titulaire: MEDINOV SA, F-42312 Roanne Cedex (FR)
(72) Inventeur: Colombier, F-42300 Roanne (FR); Delfosse, Dr., F-54000 Nancy (FR); Moati, Jean-Claude, Dr., F-92130 Issy-les-Moulineaux (FR); Chatelet, Jean Christophe, Dr., F-69007 Lyon (FR); Ferreira André, Dr., F-69300 Caluire (FR); Brugère Pierre, Dr., F-51300 Vitry Le François (FR); Charpenet, Dr., F-88150 Thaon Les Vosges (FR); Paulin, Dr., F-52000 Chaumont (FR); Ginefri Jean-Paul, Dr., Fontaine F-21121 Fontaine-Les-Dijon (FR); Préaut, Dr., F-55000 Bar-Le-Duc (FR)
(74) Mandataire: Dupuis, François

(56) Documents cités:
- EP-A- 0 069 683
- EP-A- 0 186 471
- EP-A- 0 194 326
- EP-A- 0 336 774
- EP-A- 0 381 352

## Description

L'invention concerne plus particulièrement les prothèses du type de celle comprenant un implant fémoral et un implant tibial non liés pour constituer une prothèse du genou dite à glissement.

D'une manière connue, l'implant fémoral présente deux patins condyliens réunis par une partie commune constituant ou non une trochlée. Les patins présentent un profil externe courbe et déterminé pour coopérer en appui et à glissement sur un plateau que présente l'élément tibial. Le plateau, généralement réalisé en polyéthylène, est fixé dans une embase métallique. L'élément fémoral et l'élément tibial présentent tout agencement pour leur ancrage et fixation, respectivement au niveau des têtes fémorale et tibiale des os.

Il existe sur le marché, de nombreuses prothèses du genou, du type de celle décrite ci-dessus. Ces prothèses sont généralement utilisées, lorsqu'au moins les deux ligaments croisés sont conservés ou bien lorsque soit le ligament antérieur ou le ligament croisé postérieur est conservé.

Compte-tenu de l'absence de certains des ligaments, les éléments de prothèse doivent présenter des agencements pour éviter tout phénomène de luxation et assurer la stabilité des éléments, notamment lors du mouvement de flexion - extension.

Généralement pour résoudre ces problèmes de stabilité, les plateaux tibiaux sont profilés pour éviter ce phénomène de luxation par rapport au condyle.

Très souvent, lorsque le ligament antérieur est sectionné, ce qui représente 90 % des cas, le profil particulier du plateau tibial est réalisé par un rebord postérieur. Toutefois, ce rebord limite l'amplitude de la prothèse et, en cas de mauvaise implantation, peut entrainer des descellements de l'élément tibial par un "baillement" antérieur, compte-tenu des contraintes qu'il subit.

Lorsque le ligament postérieur est détruit, la stabilisation antérieure est assurée, soit par un rebord antérieur formé dans le plateau tibial, soit par une prohéminence profilée, sous forme de condyle, formée entre les deux patins condyliens. Là encore, cette solution ne donne pas entière satisfaction. Dans l'un et l'autre cas, il en résulte une amplitude limitée et un effet de tiroir antérieur, lorsque le patient passe de la position fléchie à la position d'extension, levée chaise par exemple.

On peut citer également l'enseignement du brevet EP-A-0381352 qui décrit une prothèse du genou dont l'élément tibial présente, dans sa partie médiane, une prohéminence coopérant en position de flexion avec un élément de butée formé transversalement dans l'échancrure intracondylienne de l'élément fémoral. De telles dispositions ne permettent pas d'assurer le blocage vers l'arrière de l'élément fémoral, ce qui s'avère important lorsque les ligaments sont détruits.

Enfin, selon l'état de la technique, il est nécessaire d'avoir, en fonction du cas pathologique à traiter, notamment en fonction du ou des ligaments qui sont détruits, au moins deux types de prothèses de formes différentes, ce qui nécessite un stock d'implants et d'ancillaires important. Or, c'est seulement au moment de l'intervention, que le chirurgien voit réellement l'état ligamentaire du patient.

L'invention s'est fixée pour but de remédier à ces inconvénients, de manière, simple, sûre, efficace et rationnelle.

Le problème que se propose de résoudre l'invention, est d'assurer, dans le cas d'une prothèse du genou, d'une part, la stabilisation du condyle fémoral lors du mouvement flexion - extension et, d'autre part, assurer un blocage antéro-postérieur quand les deux ligaments sont détruits ou bien obtenir une butée postérieure ou antérieure et ce, quel que soit l'état ligamentaire du patient, en ayant pour objectif d'avoir un seul type de prothèse.

Pour résoudre un tel problème, il a été conçu et mis au point, une prothèse totale du genou du type à glissement, comprenant de manière connue, un élément fémoral dont les patins condyliens coopèrent en appui sur un plateau d'un élément tibial, les éléments tibial et fémoral présentant en combinaison, des moyens complémentaires de butée et de guidage. Selon l'invention, pour résoudre le problème posé, il est revendiqué que les moyens comprennent une cale profilée démontable, montée avec capacité de réglage en translation dans un organe support solidaire de l'élément tibial, ladite cale étant convenablement profilée pour coopérer en butée avec au moins un doigt solidaire d'une partie de l'élément fémoral, en vue d'assurer la stabilisation des deux éléments en respectant l'isométrie ligamentaire quand le ligament antérieur ou les deux ligaments croisés sont détruits.

Compte-tenu de ces caractéristiques et de la stabilisation obtenue entre l'élément fémoral et l'élément tibial :
- la surface d'appui et de glissement du plateau tibial est entièrement plane.
- le condyle externe présente un rayon d'enroulement plus grand.
- l'arc d'enroulement des deux condyles est allongé respectant la longueur anatomique des condyles fémoraux.
- l'embase tibiale est asmétrique pour améliorer l'assise de l'implant sur les cortycales.

Pour tenir compte du mouvement anatomique de flexion, la cale présente au moins un rayon de courbure déterminé en fonction des mouvements de flexion, ledit rayon constituant une surface de glissement coopérant avec le doigt de l'élément fémoral.

Dans une autre forme de réalisation, la cale forme une échancrure profilée ouverte dans laquelle est engagé à glissement, le doigt de l'élément fémoral, le profil de l'échancrure étant déterminé en fonction des mouvements de flexion.

La cale est disposée au niveau du massif des épines tibiales.

Le doigt de butée de l'élément fémoral déborde dans l'échancrure, délimitée par les patins condyliens.

Pour résoudre le problème posé de changer à volonté la cale, l'organe support est engagé dans une ouverture formée dans l'épaisseur du plateau tibial, en y étant maintenu par des organes de fixation.

Pour résoudre le problème posé d'assurer, d'une part, l'interchangeabilité de la cale et, d'autre part, son réglage en translation, l'organe support présente une rainure débouchante pour le coulissement et le guidage de la cale et présente transversalement une pluralité de trous débouchants, aptes à recevoir un axe de fixation engagé librement dans l'épaisseur de la cale, en vue de faire varier sa position.

Les extrémités de l'axe coopèrent avec les rebords de l'ouverture du plateau assurant ainsi le blocage en translation de l'axe.

L'invention est exposée, ci-après plus en détail à l'aide des dessins annexés, dans lesquels :
La figure 1 est une vue en perspective des éléments de la prothèse avant montage.
La figure 2 est une vue en perspective correspondant à la figure 1 après montage des éléments.
La figure 3 est une vue en coupe longitudinale montrant le positionnement de l'élément fémoral sur l'élément tibial en position d'extension.
La figure 4 est une vue correspondant à la figure 3 en position de flexion.
La figure 5 est une vue en coupe longitudinale correspondant à la figure 3 montrant une autre forme de réalisation de la cale, dans le cas où les ligaments antérieurs et postérieurs sont détruits.
La figure 6 est une vue en plan de l'élément tibial.
La figure 7 est une vue en coupe transversale considérée selon la ligne 7.7 de la figure 6.
Les figures 8 et 9 sont des vues à caractère purement schématique montrant des exemples de réglage en hauteur de la cale.

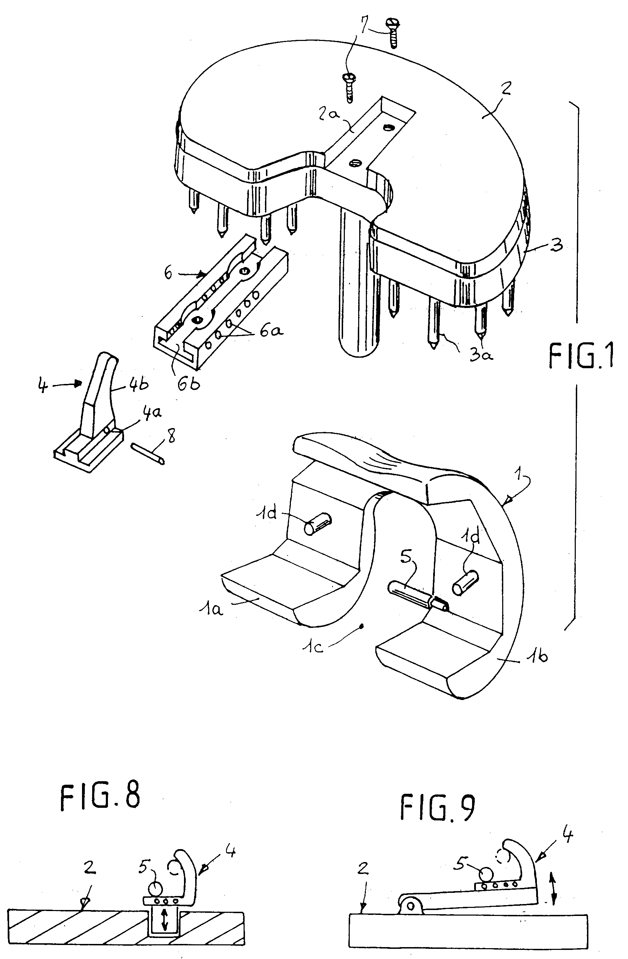

Comme le montre la figure 1, la prothèse comporte de manière connue, un élément fémoral (1) dont les patins condyliens (1a) et (1b) coopèrent en appui, sur un plateau (2) d'un élément tibial. Le plateau (2) est en polyéthylène et est fixé par tout moyen connu et approprié sur une embase métallique (3). L'élément fémoral (1) et l'embase tibiale (3) présente tout agencement (1d) et (3a) pour leur ancrage au niveau des parties d'os correspondantes.

Selon une caractéristique à la base de l'invention, les éléments tibial et fémoral présentent en combinaison, des moyens complémentaires de butée et de guidage aptes à assurer leur stabilisation, notamment le blocage vers l'arrière de l'élément fémoral ou de l'élément tibial, quand le ligament antérieur est détruit et un blocage antéro-postérieur, quand les deux ligaments sont détruits.

Dans ce but, le plateau tibial (2) reçoit, avec capacité de réglage en translation et de manière démontable, une cale profilée (4). Cette cale (4) est convenablement profilée pour coopérer en butée avec au moins un doigt (5) solidaire d'une partie de l'élément fémoral (1).

Comme le montre la figure 1, la cale (4) est engagée à libre coulissement, dans un organe support (6) solidaire du plateau tibial (2). L'organe support (6) présente une rainure débouchante (6b) de section transversale en forme de T renversé, pour l'engagement, à libre coulissement, de la base (4a) de la cale (4) qui présente une section complémentaire. L'organe (6) est intégré dans l'épaisseur du plateau tibial (2).

Par exemple, l'organe (6) est engagé dans une ouverture (2a) de forme complémentaire du plateau et est fixé par vis (7) dans l'épaisseur de l'embase tibiale (3). Le blocage en translation de la cale (4) dans l'organe (6) est assuré par un axe transversal (8) susceptible d'être engagé successivement dans une pluralité de trous (6a) formés dans l'épaisseur des branches de l'organe (6).

Après avoir positionné la cale (4) dans l'organe (6), il suffit d'engager l'axe (8) dans l'un des trous (6a), le blocage en translation de cet axe est avantageusement effectué par les rebords de l'ouverture (2a) du plateau tibial (2)

La cale (4) présente au moins un rayon de courbure (4b) dont le profil est déterminé en fonction des mouvements de flexion de l'élément fémoral sur l'élément tibial. Ce rayon de courbure (4b) constitue une surface de glissement coopérant avec le doigt (5).

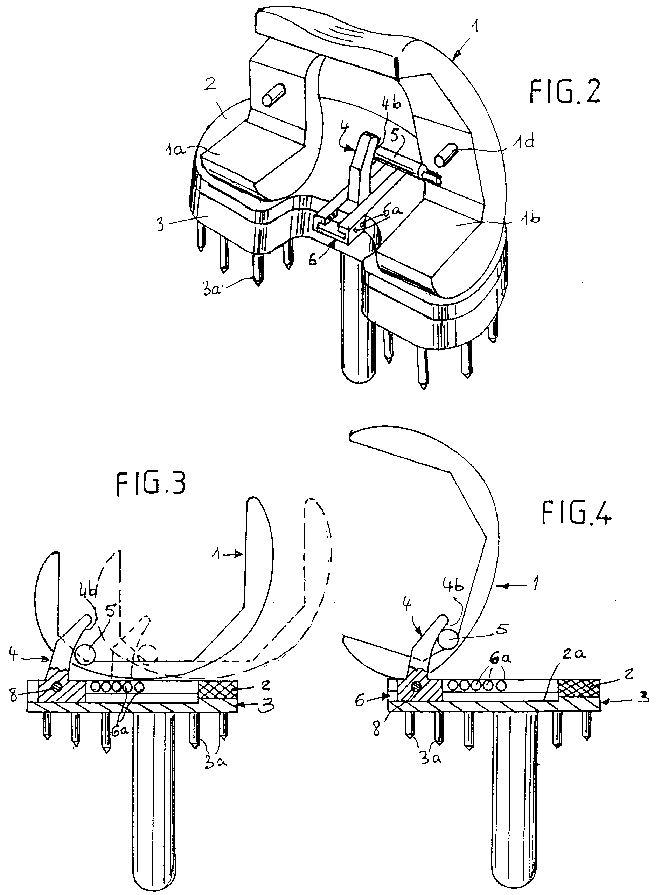

A cet effet, ce doigt est établi en débordement de l'échancrure (1c) délimitée par les patins condyliens (1a) et (1b). Comme le montrent notamment les figures 3 et 4, ces dipositions permettent d'assurer un blocage vers l'arrière de l'élément fémoral, quand le ligament est détruit.

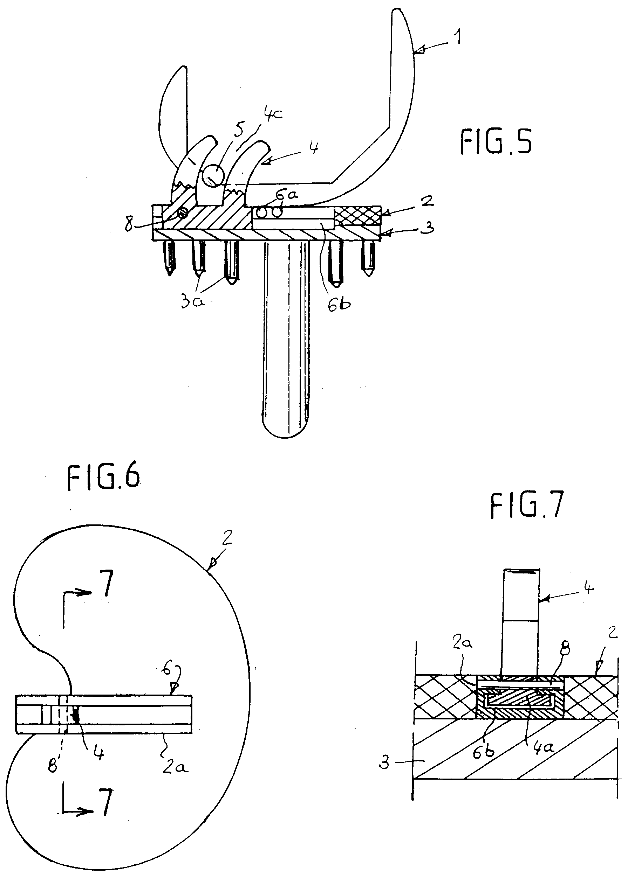

Lorsque les deux ligaments sont détruits et pour obtenir un blocage antéro-postérieur, la cale (4) présente une échancrure profilée ouverte (4c) dans laquelle est engagé à glissement, le doigt (5) de l'élément fémoral (1). Le profil de l'échancrure (4c) est déterminé, comme précédemment, en fonction des mouvements de flexion (figure 5).

La cale (4) est située au niveau du massif des épines tibiales. Cette cale est réglée par le chirurgien en per-opératoire, en fonction du test de flexion pour obtenir la flexion maximum. Le réglage en translation de la cale dans les conditions indiquées permet de respecter l'isométrie ligamentaire du genou. En outre, la cale (4) est personnalisée au cours de l'opération en fonction de l'état ligamentaire. Ce critère est très important, étant donné que le chirurgien ne connaît jamais, avant la pose de la prothèse, l'état des deux ligaments.

Selon l'invention, il suffit donc de changer la cale pour obtenir une prothèse du type postéro-stabilisée ou une prothèse du type antéro-postéro-stabilisée.

Les avantages ressortent bien de la description, en particulier on souligne et on rappelle :
- la possibilité d'adapter la prothèse en fonction de l'état ligamentaire du patient en adaptant en conséquence, la cale qui fait office de butée, évitant ainsi d'avoir un stock important d'implants et d'ancillaires.
- la simplicité de réalisation par rapport aux implants constitués d'un troisième condyle.
- la conception du plateau tibial dont la surface d'appui est entièrement plane, en opposition aux plateaux tibiaux de l'état de la technique qui présente des cupules rétentives limitant la flexion, supprimant la rotation tibiale en flexion et favorisant le descellement.

Enfin, on prévoit également de rendre réglable en hauteur, la cale (4) par rapport à l'embase tibiale. Ce réglage en hauteur peut s'effectuer par tout moyen connu et approprié.

A la figure 8, la cale est montée avec capacité de déplacement en hauteur, dans un puit ménagé dans l'épaisseur de l'embase tibiale, tandis qu'à la figure 9, la cale est montée avec capacité d'orientation angulaire par rapport l'embase tibiale.

## Revendications

1. Prothèse totale du genou du type à glissement, comprenant un élément fémoral (1) dont les patins condyliens (1a) (1b) coopèrent en appui sur un plateau (2) d'un élément tibial, les éléments tibial (2) et fémoral (1) présentant en combinaison, des moyens complémentaires (4) (5) de butée et de guidage, caractérisée en ce que les moyens comprennent une cale profilée démontable (4), montée avec capacité de réglage en translation dans un organe support (6) solidaire de l'élément tibial, ladite cale (4) étant convenablement profilée pour coopérer en butée avec au moins un doigt (5) solidaire d'une partie de l'élément fémoral (1), en vue d'assurer la stabilisation des deux éléments (2) (1) en respectant l'isométrie ligamentaire quand le ligament antérieur ou les deux ligaments croisés sont détruits.

2. Prothèse selon la revendication 1, caractérisée en ce que la surface d'appui et de glissement du plateau tibial (2) est entièrement plane.

3. Prothèse selon la revendication 1, caractérisée en ce que la cale (4) présente au moins un rayon de courbure déterminé en fonction des mouvements de flexion, ledit rayon constituant une surface de glissement coopérant avec le ou les doigts (5) de l'élément fémoral (1).

4. Prothèse selon la revendication 1, caractérisée en ce que la cale (4) forme une échancrure profilée (4c) ouverte dans laquelle est engagé à glissement, le doigt (5) de l'élément fémoral (1), le profil de l'échancrure (4c) étant déterminé en fonction des mouvements de flexion.

5. Prothèse selon la revendication 1, caractérisée en ce que la cale (4) est disposée au niveau du massif des épines tibiales.

6. Prothèse selon la revendication 1, caractérisée en ce que le doigt (5) de butée de l'élément fémoral (1) déborde dans l'échancrure (1c) délimitée par les patins condyliens (1a) (1b).

7. Prothèse selon la revendication 1, caractérisée en ce que l'organe support (6) est engagé dans une ouverture (2a) formée dans l'épaisseur du plateau tibial (2), en y étant maintenu par des organes de fixation (7).

8. Prothèse selon la revendication 1, caractérisée en ce que l'organe support (6) présente une rainure débouchante (6b) pour le coulissement et le guidage de la cale (4) et présente transversalement une pluralité de trous débouchants (6a), aptes à recevoir un axe de fixation (8) engagé librement dans l'épaisseur de la cale (4), en vue de faire varier sa position.

9. Prothèse selon la revendication 8, caractérisée en ce que les extrémités de l'axe (8) coopèrent avec les rebords de l'ouverture du plateau (2) assurant ainsi le blocage en translation de l'axe (8).

10. Prothèse selon la revendication 1, caractérisée en ce que la cale (4) est montée avec capacité de réglage en hauteur, par rapport à l'embase tibiale (2).

## Claims

1. A total knee prosthesis of the sliding type, comprising a femoral element (1), the condylar runners (1a)(1b) of which rest on the plateau (2) of a tibial element, said tibial (2) and femoral (1) elements featuring combined complementary means (4)(5) for stopping and guiding, characterised in that said means comprise a removable shaped lock means (4) so mounted as to allow translation control in a supporting body (6) fixed to the tibial element, said lock means (4) being suitably shaped so as to provide a stop in connection with at least one finger (5) protruding from one part of the femoral element (1), designed to ensure stabilisation of both elements (2)(1) and ensure ligament isometry when the anterior ligament or both cruciate ligaments are lost.

2. A prosthesis as claimed in to claim 1, characterised in that the supporting and sliding surface of said tibial plateau (2) is entirely flat.

3. A prosthesis as claimed in claim 1, characterised in that said lock means (4) has at least one radius of curvature determined according to the flexion movements, said radius constituting a sliding surface co-operating with one or several fingers (5) of the femoral element (1).

4. A prosthesis as claimed in claim 1, characterised in that said lock means (4) has a shaped open groove (4c) in which the finger (5) of the femoral element (1) is slidably engaged, the profile of said groove (4c) being determined according to flexion movements.

5. A prosthesis as claimed in claim 1, characterised in that said lock means (4) is located at the level of the tibial spines.

6. A prosthesis as claimed in claim 1, characterised in that said stop finger (5) of the femoral element (1) protrudes into the gully (1c) bounded by the condylar runners (1a)(1b).

7. A prosthesis as claimed in claim 1, characterised in that the supporting body (6) fits into an opening (2a) shaped in the thickness of the tibial plateau (2), where it is fastened by fastening means (7).

8. A prosthesis as claimed in claim 1, characterised in that the supporting body (6) exhibits an open groove (6b) for sliding and guiding said lock means (4) and, transversally, a plurality of through holes (6a) adapted to receive a fixation rod (8) freely inserted into the thickness of said lock means (4) and designed to allow variation of the latter's position.

9. A prosthesis as claimed in claim 8, characterised in that the ends of said rod (8) co-operate with the edges of the groove in the plateau (2), thus ensuring translation locking of said fixation rod (8).

10. A prosthesis as claimed in claim 1, characterised in that said lock means (4) is mounted so as to allow height adjustment relative to said tibial base (2).

## Patentansprüche

1. Totalknie-Gleitprothese mit einem femoralen Teil (1), dessen Kondylenschuhe (1a) (1b) in Auflage auf einem Plateau (2) eines tibialen Elements zusammenwirken, wobei das tibiale (2) und das femorale (1) Element sich ergänzende kombinierte Anschlags- und Führungsvorrichtungen (4) (5) aufweisen, dadurch gekennzeichnet, daß die Vorrichtungen einen abnehmbaren profilierten Keil (4) umfassen, der in der Translationsbewegung verstellbar in einem Auflageteil (6) angebracht ist, das mit dem tibialen Element fest verbunden ist, wobei der Keil (4) entsprechend profiliert ist, um im Anschlag mit mindestens einem Stift (5) zusammenzuwirken, der mit einem Teil des femoralen Elements (1) fest verbunden ist, um unter Beachtung der Bandisometrie für die Stabilisierung der beiden Elemente (2) (1) zu sorgen, wenn das Vorderband bzw. die beiden Kreuzbänder zerstört sind.

2. Prothese nach Anspruch 1, dadurch gekennzeichnet, daß die Auflage- und Gleitfläche des Tibiaplateaus (2) vollkommen eben ist.

3. Prothese nach Anspruch 1, dadurch gekennzeichnet, daß der Keil (4) mindestens einen in Abhängigkeit von den Beugebewegungen festgelegten Krümmungsradius aufweist, wobei dieser Radius eine Gleitfläche bildet, die mit dem (den) Finger(n) (5) des femoralen Elements (1) zusammenwirkt.

4. Prothese nach Anspruch 1, dadurch gekennzeichnet, daß der Keil (4) eine offene profilierte Einbuchtung (4c) bildet, in die der Stift (5) des femoralen Elements (1) gleitend eingeführt ist, wobei das Profil der Einbuchtung (4c) von den Beugebewegungen abhängig ist.

5. Prothese nach Anspruch 1, dadurch gekennzeichnet, daß der Keil (4) auf Höhe des Schienbeindornmassivs angeordnet ist.

6. Prothese nach Anspruch 1, dadurch gekennzeichnet, daß der Anschlagstift (5) des femoralen Elements (1) in die von den Kondylenschuhen (1a) (1b) begrenzte Einbuchtung (1c) hineinragt.

7. Prothese nach Anspruch 1, dadurch gekennzeichnet, daß das Auflageteil (6) in eine Öffnung (2a) im Tibiaplateau (2) eingeführt ist und dort durch Befestigungsorgane (7) festgehalten wird.

8. Prothese nach Anspruch 1, dadurch gekennzeichnet, daß das Auflageteil (6) eine ausführende Rille (6b) für das Gleiten und Führen des Keils (4) sowie quer dazu mehrere ausführende Bohrungen (6a) aufweist, die geeignet sind, einen Befestigungsbolzen (8) aufzunehmen, der frei beweglich in den Keil (4) eingeführt wird, um dessen Position zu ändern.

9. Prothese nach Anspruch 8, dadurch gekennzeichnet, daß die Enden des Achsbolzens (8) mit den überstehenden Kanten der Öffnung des Plateaus (2) zusammenwirken und dadurch für die Blockierung des Achsbolzens (8) in der Translationsbewegung sorgen.

10. Prothese nach Anspruch 1, dadurch gekennzeichnet, daß der Keil (4) gegenüber dem Tibiaplateau (2) in der Höhe verstellbar angebracht ist.
